# EUROPEAN PATENT APPLICATION

(11) **EP 4 737 447 A1**
(43) Date of publication of application: **06.05.2026**
(21) Application number: 24830964.3
(22) Date of filing: 28.06.2024
(51) Int. Cl.: C07D 401/04, A61K 31/4725, A61P 9/12, A61P 13/12

(54) **CRYSTAL FORM OF BAXDROSTAT, AND PREPARATION METHOD THEREFOR AND USE THEREOF**

(30) Priority: 29.06.2023 CN 202310782860
(71) Applicant: Crystal Pharmaceutical (Suzhou) Co., Ltd., Suzhou, Jiangsu 215123 (CN)
(72) Inventor: ZHAI, Xiaoting, Suzhou, Jiangsu 215123 (CN); MENG, Liping, Suzhou, Jiangsu 215123 (CN)
(74) Representative: dompatent
(86) International application number: PCT/CN2024/102325
(87) International publication number: WO 2025/002335

(57) **Abstract**

A new crystal form of Baxdrostat, and a preparation method therefor, a pharmaceutical composition containing the crystal form, and the use of the crystal form in the preparation of an aldosterone synthase inhibitor drug and a drug for treating hypertension, primary aldosteronism, and chronic kidney disease.

## Description

### TECHNICAL FIELD

The present disclosure pertains to the field of chemical crystallography, particularly relates to crystalline form of Baxdrostat, preparation method and use thereof.

### BACKGROUND

Baxdrostat is an aldosterone synthase inhibitor developed by CinCor, and is used for the treatment of hypertension, primary aldosteronism and chronic kidney disease. Positive results have been achieved in phase II clinical trial for resistant hypertension.

The chemical name of Baxdrostat is (+)-(R)-N-(4-(1-methyl-2-oxo-1,2,3,4-tetrahydroquinolin-6-yl)-5,6,7,8-tetrahydroisoquinolin-8-yl)propionamide (hereinafter referred to as Compound I), and the structural formula is as follows:

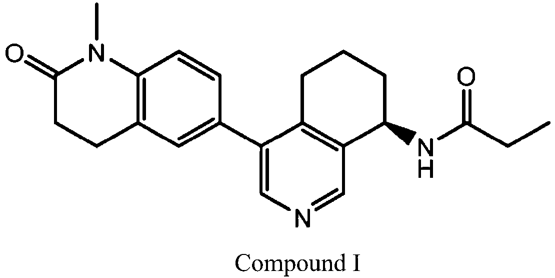

A crystalline form is a solid material whose constituents are arranged in a highly ordered microscopic structure, forming a crystal lattice that extends in all directions. Polymorphism refers to the phenomenon that a compound exists in more than one crystalline form. Compounds may exist in one or more crystalline forms, but their existence and characteristics cannot be predicted with any certainty. Different crystalline forms of active pharmaceutical ingredient (API) have different physicochemical properties, which can affect drug's in vivo dissolution and absorption and will further affect drug's clinical efficacy and safety to some extent. In particular, for some poorly soluble oral solid or semi-solid dosage forms, crystalline forms can be crucial to the performance of drug products. In addition, the physiochemical properties of a crystalline form, such as fluidity, compressibility, and grinding stability, are very important to the manufacturing process. Therefore, polymorphism is an important part of drug research and drug quality control.

WO2013041591A1 discloses the synthesis method of Compound I, but does not disclose a crystalline form of Compound I.

The inventors of the present disclosure surprisingly discovered a crystalline form of Compound I, which have advantages in hygroscopicity and stability, and is of great significance for the development of drugs containing Compound I.

### SUMMARY

The present disclosure is to provide a crystalline form of Compound I, preparation method and pharmaceutical compositions comprising the crystalline form.

According to the objective of the present disclosure, a crystalline form of Compound I is provided.

According to the objective of the present disclosure, a hydrate of Compound I is provided.

According to the objective of the present disclosure, crystalline form CSI of Compound I is provided (hereinafter referred to as Form CSI).

The X-ray powder diffraction pattern of Form CSI comprises one or two or three characteristic peaks at 2theta values of 8.1°±0.2°, 14.3°±0.2° and 21.7°±0.2°. Preferably, the X-ray powder diffraction pattern of Form CSI comprises characteristic peaks at 2theta values of 8.1°±0.2°, 14.3°±0.2° and 21.7°±0.2° using CuKα radiation.

The X-ray powder diffraction pattern of Form CSI comprises one or two or three characteristic peaks at 2theta values of 15.9°±0.2°, 18.9°±0.2° and 24.8°±0.2°. Preferably, the X-ray powder diffraction pattern of Form CSI comprises characteristic peaks at 2theta values of 15.9°±0.2°, 18.9°±0.2° and 24.8°±0.2° using CuKα radiation.

The X-ray powder diffraction pattern of Form CSI comprises one or two or three or four characteristic peaks at 2theta values of 14.7°±0.2°, 17.4°±0.2°, 18.5°±0.2° and 22.0°±0.2°. Preferably, the X-ray powder diffraction pattern of Form CSI comprises characteristic peaks at 2theta values of 14.7°±0.2°, 17.4°±0.2°, 18.5°±0.2° and 22.0°±0.2° using CuKα radiation.

The X-ray powder diffraction pattern of Form CSI comprises one or two or three or four or five or six or seven or eight or nine or ten or eleven or twelve or thirteen or fourteen or fifteen or sixteen characteristic peaks at 2theta values of 8.1°±0.2°, 14.3°±0.2°, 21.7°±0.2°, 15.9°±0.2°, 18.9°±0.2°, 24.8°±0.2°, 14.7°±0.2°, 17.4°±0.2°, 18.5°±0.2°, 22.0°±0.2°, 12.4°±0.2°, 16.3°±0.2°, 16.8°±0.2°, 20.1°±0.2°, 21.1°±0.2° and 24.5°±0.2°.

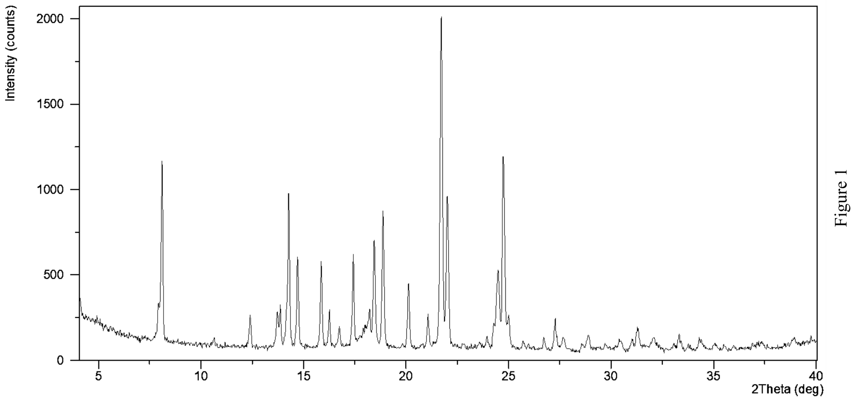

Without any limitation being implied, the XRPD pattern of Form CSI is substantially as depicted in Figure 1 using CuKα radiation.

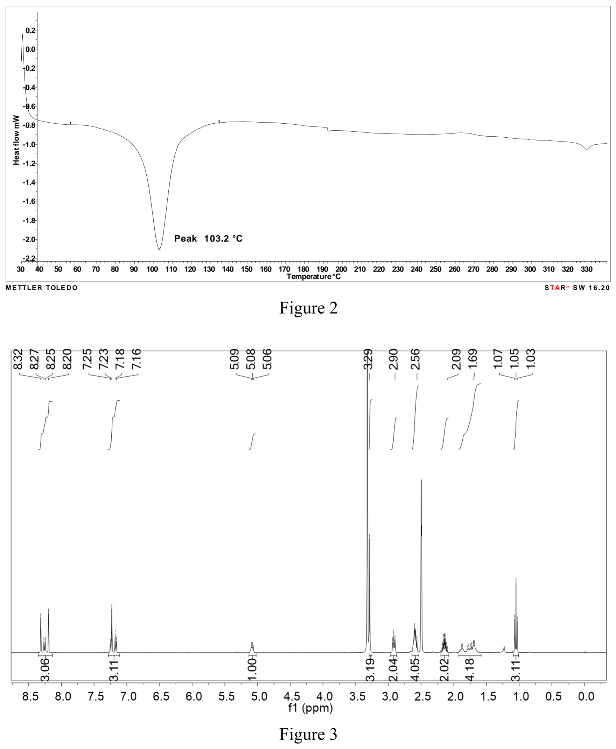

Without any limitation being implied, the DSC curve of Form CSI is substantially as depicted in Figure 2. There is an endothermic peak near 103 °C, which is the dehydration signal of Form CSI.

Without any limitation being implied, Form CSI is a hydrate.

Without any limitation being implied, Form CSI shows about 3%~6% weight loss when heated to 150 °C by TGA.

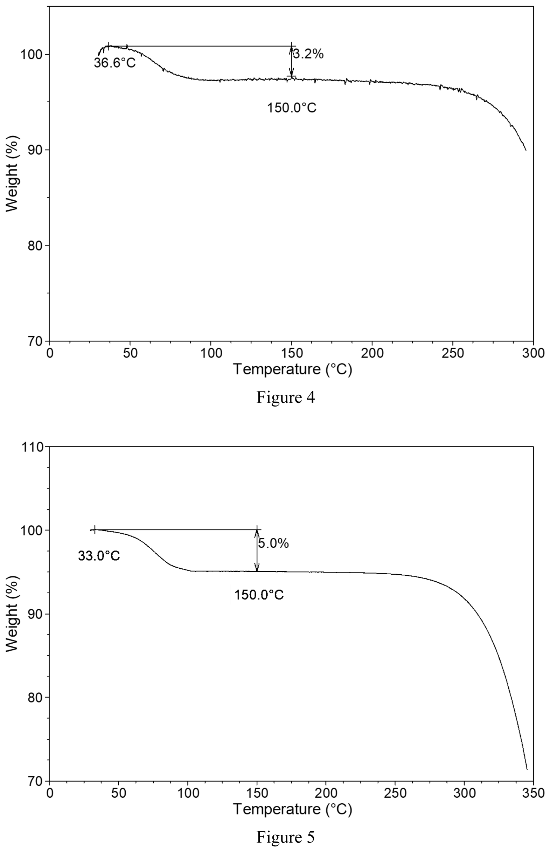

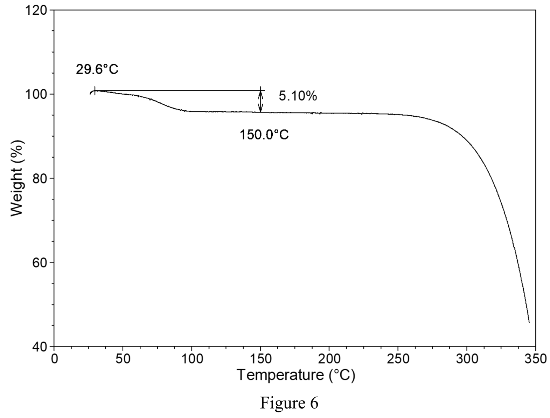

Without any limitation being implied, the TGA curves of Form CSI are substantially as depicted in Figure 4, Figure 5 and Figure 6, which show 3.2%, 5.0% and 5.1% weight loss when heated to 150 °C, respectively.

According to the objective of the present disclosure, a process for preparing Form CSI is also provided. The process comprises: Placing Compound I in a mixed solvent of methanol and methyl tert-butyl ether, stirring, and separating to obtain Form CSI.

According to the objective of the present disclosure, a pharmaceutical composition is provided, said pharmaceutical composition comprises a therapeutically effective amount of crystalline form of Compound I provided by the present disclosure and pharmaceutically acceptable excipients.

According to the objective of the present disclosure, a crystalline form of Compound I provided by the present disclosure can be used for preparing aldosterone synthase inhibitors drugs.

According to the objective of the present disclosure, a crystalline form of Compound I provided by the present disclosure can be used for preparing drugs treating hypertension, primary aldosteronism and chronic kidney disease.

### BRIEF DESCRIPTION OF THE DRAWINGS

Figure 1 shows an XRPD pattern of Form CSI
Figure 2 shows a DSC curve of Form CSI
Figure 3 shows an ¹H NMR spectrum of Form CSI
Figure 4 shows a TGA curve of Form CSI
Figure 5 shows a TGA curve of Form CSI
Figure 6 shows a TGA curve of Form CSI
Figure 7 shows an XRPD pattern overlay of Form CSI before and after storage with sealed package (from top to bottom: initial, 25 °C/60%RH for six months, 40 °C/75%RH for six months, 60 °C/75%RH for one month.)
Figure 8 shows an XRPD pattern overlay of Form CSI before and after storage with open package (from top to bottom: initial, 25 °C/60%RH for six months, 40 °C/75%RH for six months, 60 °C/75%RH for one month.)
Figure 9 shows an XRPD pattern overlay of Form CSI before and after wet ball milling (from top to bottom: initial, after wet ball milling in ethanol, after wet ball milling in water.)
Figure 10 shows a DVS plot of Form CSI
Figure 11 shows an XRPD pattern overlay of Form CSI before and after DVS test (from top to bottom: before DVS test, after DVS test.)
Figure 12 is an image showing the experimental phenomena for Example 5 (left: the phenomenon after the addition of n-pentane, right: the phenomenon after standing for a period.)

### DETAILED DESCRIPTION

The present disclosure is further illustrated by the following examples which describe the preparation and use of the crystalline form of the present disclosure in detail. It is obvious to those skilled in the art that changes in the materials and methods can be accomplished without departing from the scope of the present disclosure.

The abbreviations used in the present disclosure are explained as follows:
XRPD: X-ray Powder Diffraction
¹H NMR: Proton Nuclear Magnetic Resonance
HPLC: High Performance Liquid Chromatography
DSC: Differential Scanning Calorimetry
TGA: Thermo Gravimetric Analysis
DVS: Dynamic Vapor Sorption
RH: Relative Humidity

Instruments and methods used for data collection:
XRPD patterns in the present disclosure were acquired by a Bruker D8 DISCOVER X-ray powder diffractometer. The parameters of the X-ray powder diffraction method of the present disclosure are as follows:
X-Ray source: Cu, Kα
Kα1 (Å): 1.54060; Kα2 (Å): 1.54439
Kα2/Kα1 intensity ratio: 0.50
Voltage: 40 kV
Current: 40 mA
Scan range (20): from 4.0 degree to 40.0 degree

DSC data in the present disclosure were acquired by METTLER TOLEDO DSC 3. The parameters of the DSC method of the present disclosure are as follows:
Heating rate: 10 °C/min
Purge gas: N₂

¹H NMR data were collected from a Bruker Avance II DMX 400M HZ NMR spectrometer. 1-5 mg of sample was weighed and dissolved with 0.5 mL of deuterated dimethyl sulfoxide to obtain a solution with a concentration of 2-10 mg/mL.

TGA data in the present disclosure were acquired by a TA Q500. The parameters of the TGA method of the present disclosure are as follows:
Heating rate: 10 °C/ min

Purge gas: N₂DVS data in the present disclosure were measured via an SMS (Surface Measurement Systems Ltd.) intrinsic DVS instrument. The instrument control software is DVS-Intrinsic control software. Typical parameters for DVS test are as follows:
Temperature: 25 °C
Gas and flow rate: N₂, 200 mL/min
RH range: 0%RH to 95%RH

The parameters for related substance detection in the present disclosure are shown in Table 1.

**Table 1**

| | | |
|---|---|---|
| Instrument | Agilent 1260 | |
| Column | Waters XBridge C18, 4.6 mm×150 mm, 5 µm | |
| Mobile phase | A: 0.1% H₃PO₄ in H₂O (pH3.0, NaOH) | |
| | B: Acetonitrile | |
| Gradient | Time (min) | %B |
| | 0.0 | 8 |
| | 20.0 | 45 |
| | 23.0 | 80 |
| | 25.0 | 80 |
| | 25.1 | 8 |
| | 30.0 | 8 |
| Flow rate | 1.0 mL/min | |
| Injection volume | 5 µL | |
| Detector wavelength | 210 nm | |
| Column temperature | 42 °C | |
| Sample temperature | Room temperature | |
| Diluent | Acetonitrile: H₂O = 50:50 (v/v) | |

In the present disclosure, said "crystalline form" refers to a solid substance that exhibits different molecular arrangements and/or conformations within the crystal lattice.

Said "room temperature" is not a specific temperature, but a temperature range of 10-30 °C.

Said "characteristic peak" refers to a representative diffraction peak used to distinguish crystals, which usually can have a deviation of ±0.2° using CuKα radiation.

In the present disclosure, said "stirring" is accomplished by using a conventional method in the field such as magnetic stirring or mechanical stirring and the stirring speed is 50 to 1800 r/min. Preferably, the magnetic stirring speed is 300 to 900 r/min, and mechanical stirring speed is 100 to 300 r/min.

In the present disclosure, said "separation" is accomplished by using a conventional method in the field such as centrifugation or filtration. The operation of "centrifugation" is as follows: the sample to be separated is placed into the centrifuge tube, and then centrifuged at a rate of 10000 r/min until the solid all sink to the bottom of the tube.

In the present disclosure, "crystal" or "crystalline form" can be identified by the X-ray diffraction pattern shown herein. Those skilled in the art can understand that the X-ray powder diffraction pattern depends on the instrument conditions, the sample preparation and the purity of samples. The relative intensity of the diffraction peaks in the X-ray diffraction pattern may also vary with the experimental conditions. Therefore, the order of the diffraction peak intensities cannot be regarded as the sole or decisive factor to determine the crystalline form. In fact, the relative intensity of the diffraction peaks in the X-ray powder diffraction pattern is related to the preferred orientation of the crystals, and the diffraction peak intensities shown herein are illustrative and identical diffraction peak intensities are not required. Thus, it will be understood by those skilled in the art that a crystalline form of the present disclosure is not necessarily to have exactly the same X-ray diffraction pattern of the example shown herein. Any crystalline forms whose X-ray diffraction patterns have the same or similar characteristic peaks should be within the scope of the present disclosure. Those skilled in the art can compare the patterns shown in the present disclosure with that of an unknown crystalline form to identify whether these two groups of patterns reflect the same or different crystalline forms.

In some embodiments, Form CSI of the present disclosure is pure and substantially free of any other crystalline forms. In the present disclosure, the term "substantially free" when used to describe a novel crystalline form, it means that the content of other crystalline forms in the novel crystalline form is less than 20% (w/w), specifically less than 10% (w/w), more specifically less than 5% (w/w) and furthermore specifically less than 1% (w/w).

In the present disclosure, the term "about" when referring to a measurable value such as weight, time, temperature, and the like, is meant to encompass variations of ± 10%, ± 5%, ± 1%, ± 0.5%, or even ± 0.1% of the specified amount.

Unless otherwise specified, the following examples were conducted at room temperature.

According to the present disclosure, Compound I used as raw materials include, but are not limited to solid (crystal and amorphous), oil, liquid form or solution.

Unless otherwise specified, raw materials of Compound I used in the following examples can be prepared by the method described in the document WO2013041591A1 or obtained through commercial routes.

### Example 1

14.6 mg of Compound I was weighed into a glass vial, and 0.2 mL of a mixed solvent of methanol and methyl tert-butyl ether (1:19, v/v) was added. The mixture was then stirred at room temperature for 3 days, followed by centrifugation and separation. The solid was obtained by vacuum drying at room temperature. After detection, the obtained solid is confirmed to be Form CSI of the present disclosure. The XRPD data are presented in Table 2, and the XRPD pattern is substantially as depicted in Figure 1.

The DSC curve of Form CSI is substantially as depicted in Figure 2, which shows an endothermic peak at around 103 °C, corresponding to the dehydration signal of Form CSI.

The ¹H NMR spectrum of Form CSI is substantially as depicted in Figure 3. The observed peaks are consistent with the structure of Compound I, indicating that no organic solvent residue is present in Form CSI.

**Table 2**

| 2Theta (°) | d space (Å) | Relative intensity (%) |
|---|---|---|
| 8.1 | 10.9 | 53.8 |
| 10.6 | 8.3 | 1.6 |
| 12.4 | 7.1 | 9.6 |
| 13.7 | 6.5 | 10.7 |
| 13.9 | 6.4 | 12.9 |
| 14.3 | 6.2 | 46.4 |
| 14.7 | 6.0 | 27.2 |
| 15.9 | 5.6 | 25.9 |
| 16.3 | 5.5 | 11.5 |
| 16.8 | 5.3 | 6.5 |
| 17.4 | 5.1 | 27.7 |
| 18.2 | 4.9 | 11.9 |
| 18.5 | 4.8 | 33.0 |
| 18.9 | 4.7 | 41.1 |
| 20.1 | 4.4 | 19.4 |
| 21.1 | 4.2 | 9.8 |
| 21.7 | 4.1 | 100.0 |
| 22.0 | 4.0 | 45.6 |
| 24.0 | 3.7 | 3.5 |
| 24.5 | 3.6 | 23.8 |
| 24.8 | 3.6 | 57.8 |
| 25.0 | 3.6 | 10.3 |
| 25.7 | 3.5 | 2.3 |
| 26.7 | 3.3 | 3.2 |
| 27.3 | 3.3 | 7.9 |
| 27.7 | 3.2 | 3.8 |
| 28.9 | 3.1 | 4.5 |
| 30.4 | 2.9 | 2.7 |
| 31.3 | 2.9 | 5.7 |
| 32.1 | 2.8 | 3.8 |
| 33.3 | 2.7 | 4.2 |
| 34.3 | 2.6 | 2.6 |
| 35.1 | 2.6 | 1.8 |
| 37.2 | 2.4 | 1.9 |
| 38.9 | 2.3 | 3.2 |

### Example 2

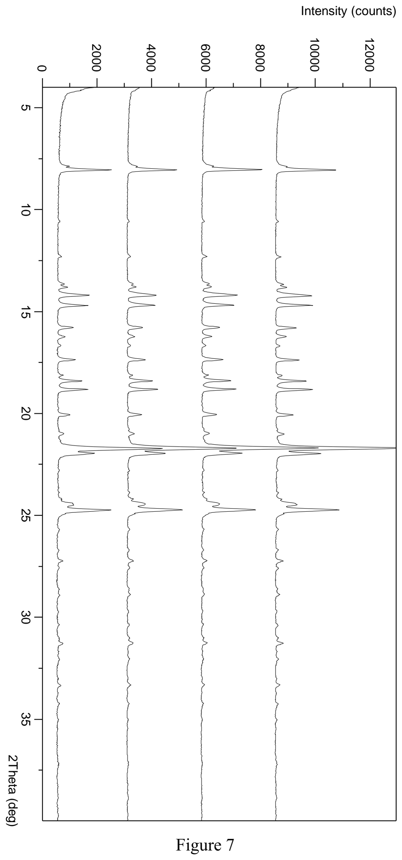

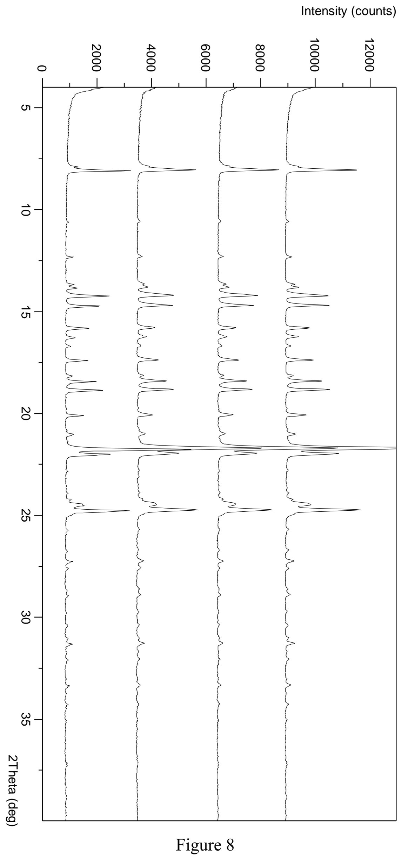

A certain amount of Form CSI prepared by the present disclosure was stored under conditions of 25 °C/60% RH, 40 °C/75% RH and 60 °C/75% RH. The purity and crystalline form were checked by HPLC and XRPD. The results are listed in Table 3, and the XRPD overlay is substantially as depicted in Figure 7 and Figure 8. The results indicate that Form CSI remains stable for at least 6 months under 25 °C/60%RH and 40 °C/75%RH conditions with its purity essentially unchanged, demonstrating excellent stability under both long-term and accelerated conditions. Under 60 °C/75%RH, it remains stable for at least 1 month with its purity also essentially unchanged, indicating very good stability even under stress conditions.

**Table 3**

| Condition | Packing conditions | Time | Solid form | Purity |
|---|---|---|---|---|
| Initial | - | - | Form CSI | 99.23% |
| 25 °C/60%RH | Sealed package | 6 months | Form CSI | 99.22% |
| | Open package | 6 months | Form CSI | 99.27% |
| 40 °C/75%RH | Sealed package | 6 months | Form CSI | 99.21 % |
| | Open package | 6 months | Form CSI | 99.26% |
| 60 °C/75%RH | Sealed package | 1 month | Form CSI | 99.24% |
| | Open package | 1 month | Form CSI | 99.26% |

Form CSI API exhibits good stability under long-term conditions, which is beneficial for drug storage. Seasonal variations, climatic differences in various regions, and environmental factors such as high temperature and high humidity can affect the storage, transportation, and production of drug substance and formulations. Therefore, the stability of API under accelerated and stress conditions is crucial for a drug. Form CSI API shows good stability under stress conditions, which helps prevent adverse effects on drug quality due to polymorphic transformation or decrease in purity during storage.

### Example 3

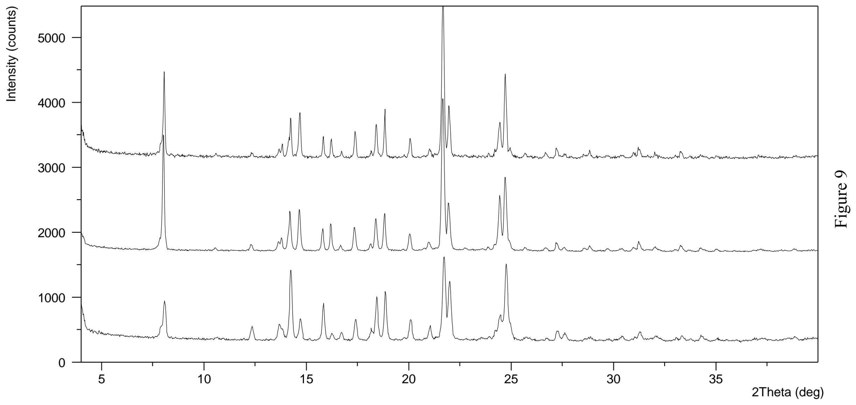

A certain amount of Form CSI solid was placed in a centrifuge tube, and about 10 µL of corresponding solvent was added inside, along with a 5 mm grinding ball. The centrifuge tube containing the sample was installed on the GT300 vibration ball mill and subjected to wet ball milling at 1800 rpm for 10 min. The experimental results are shown in Table 4 and Figure 9. The results indicate that Form CSI remains unchanged before and after wet ball milling, and no significant decrease in crystallinity is observed, demonstrating good grinding stability.

**Table 4**

| Before grinding | Solvent | After grinding |
|---|---|---|
| Form CSI | Water | Form CSI |
| | Ethanol | Form CSI |

During the formulation processing, it is often necessary to grind or crush the API. Good physical stability can reduce the risk of decreased crystallinity and polymorphic transformation of the API during the formulation process.

### Example 4

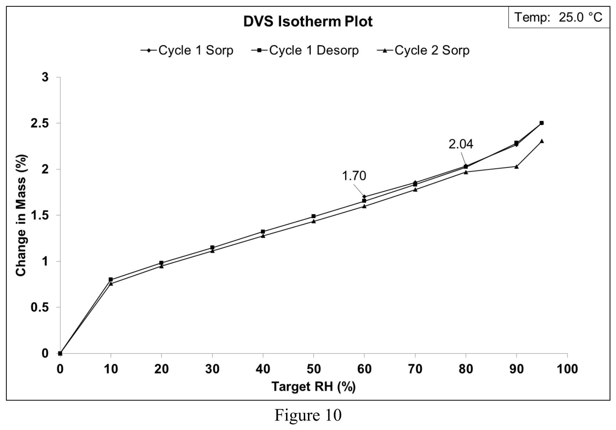

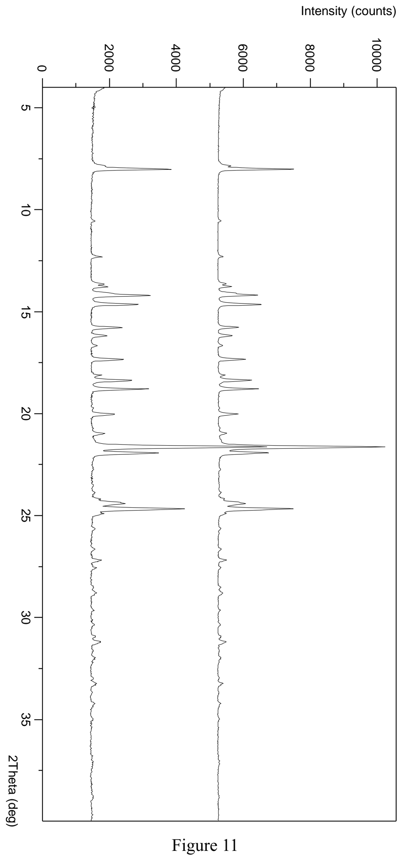

A certain amount of Form CSI was used to test the weight gain at various humidity by a DVS instrument. The weight gains at each relative humidity were recorded in a cycle of 60%RH-95%RH-0%RH-95%RH-0%RH. The DVS curve for Form CSI is shown in Figure 10, which shows a weight gain of 0.34% from 60%RH to 80%RH. The XRPD overlay before and after DVS test is substantially as depicted in Figure 11. No form change was observed for Form CSI before and after the DVS test, indicating that Form CSI has good humidity stability.

Form CSI API has good humidity stability, which helps to avoid the impact on drug quality during storage due to polymorphic transformation or purity degradation.

### Example 5

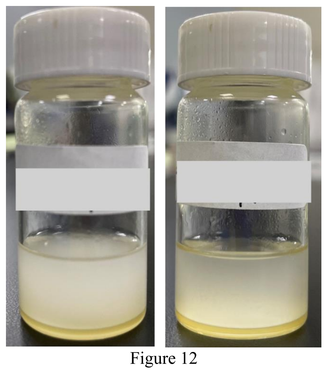

Referring to Examples 2 and 3 of WO2013041591A1, about 200 mg of Compound I was weighed into a 20 mL glass vial, followed by adding 2 mL of dichloromethane to dissolve it. Then, 5 mL of n-pentane was added at room temperature. The system became oily and phase-separated. As shown in Figure 12, no solid precipitation was observed.

The examples described above are only for illustrating the technical concepts and features of the present disclosure, and intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

## Claims

1. A crystalline form of Compound I,

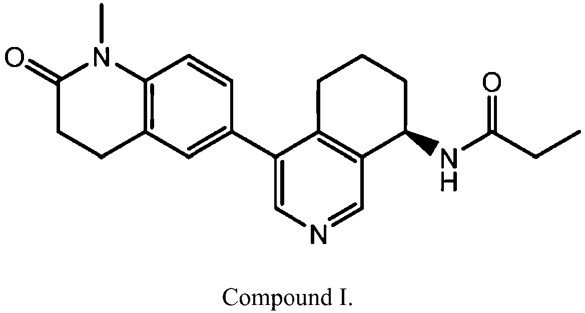

2. The crystalline form of Compound I according to claim 1, wherein the X-ray powder diffraction pattern comprises at least one characteristic peak at 2theta values of 8.1°±0.2°, 14.3°±0.2° and 21.7°±0.2° using Cu-Kα radiation.

3. The crystalline form of Compound I according to claim 1, wherein the X-ray powder diffraction pattern comprises at least one characteristic peak at 2theta values of 15.9°±0.2°, 18.9°±0.2° and 24.8°±0.2° using Cu-Kα radiation.

4. The crystalline form of Compound I according to claim 1, wherein the X-ray powder diffraction pattern comprises characteristic peak at 2theta values of 14.7°±0.2°, 17.4°±0.2°, 18.5°±0.2° and 22.0°±0.2° using Cu-Kα radiation.

5. The crystalline form of Compound I according to claim 1, wherein the X-ray powder diffraction pattern is substantially as depicted in Figure 1 using Cu-Kα radiation.

6. The crystalline form of Compound I according to claim 1, wherein it is a hydrate.

7. A pharmaceutical composition, wherein said pharmaceutical composition comprises a therapeutically effective amount of crystalline form of Compound I according to claim 1, and pharmaceutically acceptable excipients.

8. Crystalline form of Compound I according to claim 1 for use of preparing aldosterone synthase inhibitor drugs.

9. Crystalline form of Compound I according to claim 1 for use of preparing drugs treating hypertension, primary aldosteronism and chronic kidney disease.
